(19)

Europäisches
Patentamt
European
Patent Office
Office européen
des brevets

(11) **EP 1 839 658 A1**

(12) **EUROPEAN PATENT APPLICATION**

(43) Date of publication:
**03.10.2007 Bulletin 2007/40**

(51) Int Cl.:
*A61K 31/353* (2006.01)   *A61K 9/16* (2006.01)

(21) Application number: **06388023.1**

(22) Date of filing: **30.03.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Hexal A/S**
**2650 Hvidovre (DK)**

(72) Inventors:
• **Christensen, Lars Hedevang**
**2870 Dyssegaerd (DK)**

• **Schlüntz, Kirsten**
**2830 Virum (DK)**
• **Felumb, Niels Christian**
**2830 Virum (DK)**

(74) Representative: **Rasmussen, Preben et al**
**Internationalt Patent-Bureau A/S**
**Rigensgade 11**
**1316 Copenhagen K (DK)**

(54) **Pharmaceutical composition comprising micronized nebivolol**

(57) The present invention relates to a solid pharmaceutical composition, which comprises micronized Nebivolol or a pharmaceutically acceptable micronized acid addition salt thereof as the active agent, a filler, a disintegrant, a binding agent and a lubricant. The dissolution of said solid composition is at least 75 % after 45 minutes even though no wetting agent is present in the composition. The present invention also relates to a method for the manufacture of a tablet, which comprises the pharmaceutical composition and uses thereof.

EP 1 839 658 A1

**Description**

The present invention relates to a solid pharmaceutical composition, which comprises micronized Nebivolol or a pharmaceutically acceptable micronized acid addition salt thereof as the active agent, a filler, a disintegrant, a binding agent and a lubricant. The dissolution of said solid composition is at least 75 % after 45 minutes.

The present invention also relates to a method for the manufacture of a tablet, which comprises the pharmaceutical composition. Furthermore, the present invention relates to the use of said pharmaceutical composition in the manufacture of a tablet to be used as a β-adrenergic receptor-blocking drug or for use in the treatment of essential hypertension or in the treatment of chronic heart failure.

Background of invention

It is well-known within the art, that the bioavailability of an active agent after intake by a human being is very important in order for it to give rise to a pharmaceutical effect. For an oral composition, one of the important factors influencing the bioavailability is the rate of dissolution of the active compound in the gastric or intestinal fluid depending on whether the oral composition is designed to dissolve in the stomach or in the intestine. Hence, in order to examine the usability of an oral composition it is common to measure the dissolution rate of the active compound in artificial gastric or intestinal fluid as the dissolution medium.

Nebivolol, α,α'-(iminobis(methylene)bis[6-fluoro-3,4-dihydro-2H-1-benzopyran-2-methanol], and salts thereof are well known β-adrenergic receptor-blocking drugs, which are prepared as a racemic mixture of the RSSS and SRRR enantiomers. Furthermore, it is well-known that the dissolution rate of Nebivolol and salts thereof are rather low.

Nebivolol was described for the first time by Janssen Pharmaceutica N.V. in the European patent EP 0 145 067 B1, wherein a group of compounds having the general formula 2,2'-iminibisethanol and derivatives thereof was disclosed. In the description only one composition of a tablet is described in which the well-known wetting agent sodium dodecyl sulphate is incorporated. No discussion with regard to the dissolution of Nebivolol can be found in the document.

In the European patent EP 0 334 429 B1 Janssen Pharmaceutica N.V. continued their disclosure of Nebivolol by describing the use of a group of compounds including Nebivolol for the manufacture of a medicament for potentiating the effects of blood pressure reducing agents having adrenergic and/or vasodillating activity. No specific compositions are described in this document.

Solid as well as semi-solid compositions containing micronized Nebivolol or a pharmaceutical acceptable salt thereof are described in EP 0 744 946 B1. Also problems with regard to the poor dissolution rate of Nebivolol are discussed, as it is stated that oral administration of Nebivolol hydrochloride is impeded by the poor dissolution, when in a normal crystalline form. In order to solve this problem the Nebivolol hydrochloride was micronized in expectation of improving the solubility. However, the results of their experiments showed that the dissolution rate of micronized Nebivolol hydrochloride was even slower than the dissolution rate of Nebivolol hydrochloride in the normal crystalline form in the cases where no wetting agent was incorporated in the solid composition comprising the micronized Nebivolol hydrochloride. Consequently, it was concluded that a wetting agent as for example dioctylsulfosuccinate or polysorbate must be included in a solid composition in order to achieve an acceptable dissolution rate of Nebivolol hydrochloride.

Lately extensive studies have shown, however, that it is possible to prepare solid compositions containing a micronized acid addition salt of Nebivolol, which possess an improved dissolution rate without adding a wetting agent to the composition. Furthermore, experiments have shown that the smaller the particles of Nebivolol hydrochloride the faster the dissolution rate of the compound. In fact, experiments have shown that it is possible to design solid compositions having a desirable dissolution rate by controlling the particle size of Nebivolol hydrochloride.

Description of the invention

The present invention relates to a solid pharmaceutical composition comprising micronized Nebivolol or a pharmaceutically acceptable micronized acid addition salt thereof as the active agent, which is suitable for oral administration due to an excellent dissolution of the solid composition. More specifically, the present invention relates to a solid pharmaceutical composition for oral administration, said composition comprising micronized Nebivolol or a pharmaceutically acceptable micronized acid addition salt thereof as the active agent, a filler, a disintegrant, a binding agent and a lubricant, and wherein said composition has a dissolution of at least 75 % after 45 minutes. This composition is of particular interest because of its excellent dissolution in simulated gastric acid despite the fact that no wetting agent is incorporated in the solid composition. In a preferred embodiment of the invention the composition has a dissolution of at least 75 % after 10 minutes.

The solid pharmaceutical composition according to the present invention is very suitable for oral administration, because of the rather fast dissolution of the composition even though no wetting agent has been incorporated in the composition. This rather fast dissolution brings about a satisfactory bioavailability of the micronized Nebivolol in the composition when administered through the oral route.

Furthermore, it is an increasing problem that more and more people develop allergy to any one of the agents, which may be incorporated in pharmaceutical compositions or drugs. Hence, reducing the number as well as the amount of agents needed for the design of a composition such as a solid composition according to the invention will prevent or decrease the rate at which the development of allergy spreads. Consequently, the fact that no wetting agent is necessary for the design of a suitable composition comprising Nebivolol is another beneficial effect of the present invention.

The present invention also relates to a method for the manufacture of a tablet comprising a solid pharmaceutical composition according to the invention. This method is particularly suitable for the manufacture of a tablet having the chemical composition as those described in the present description.

Furthermore, the present invention relates to the use of a pharmaceutical composition according to the present invention in the manufacture of a tablet for use as a β-adrenergic receptor-blocking drug or for use in the treatment of essential hypertension or in the treatment of chronic heart failure. As discussed above, the dissolution rate of the composition makes it possible to use the composition of the present invention in the manufacture of a tablet due to the appropriate bioavailability of Nebivolol when the tablet is administered orally. Furthermore, the use of the composition according to the invention possesses the beneficial effect of reducing the risk of allergy development.

In the present description the following terminology will be used in accordance with the definitions set out below.

By the term "micronized" as used herein is meant that the solid particles of Nebivolol or its acid addition salt is broken down to small particles that are only a few micrometers in diameter. In the present description the term "volume median diameter < 10 μm", which may also be written as "D(v,0.5) < 10 μm" refers to the fact that 50% of the total amount of solid is to be found in particles having a diameter of less than 10 μm. In the same way, the term "D(v,0.9) < 40 μm" refers to the fact that 90% of the total amount of solid is to be found in particles having a diameter of less than 40 μm.

The expression "the dissolution is at least 75 % after 45 minutes" refers to the fact that at least 75 % of micronized Nebivolol or its micronized acid addition salt in the solid composition is dissolved after 45 minutes when the solid composition is subjected to the dissolution test as described below in the experimental part of this description. However, in a preferred embodiment the dissolution is at least 75 % after 10 minutes.

The invention relates to Nebivolol as the active agent. Nebivolol may be used as the free base or as a pharmaceutically acceptable acid addition salt thereof. The acid addition salt is prepared by treatment of Nebivolol with an appropriate acid. Appropriate inorganic acids include hydrochloric acid, hydrobromic acid, sulphuric acid, sulfamic acid, nitric acid, phosphoric acid and the like. And appropriate organic acids include acetic acid, propanoic acid, hydroxyacetic acid, hydroxybenzeneacetic acid, 2-hydroxypropanoic acid, 2-oxopropanoic acid, ethanedioic acid, propanedioic acid, butanedioic acid, (Z)-2-butenedioic acid, (E)-2-butenedioic acid, 2-methyl-2-butenedioic acid, 2-methylenebutanedioic acid, aminobutanedioic acid, 2-hydroxybutanedioic acid, 2,3-dihydroxybutanedioic acid, pentanedioic acid, 2-aminopentanedioic acid, 2,3,4,5,6-pentahydroxyhexanoic acid, 2-hydroxy-1,2,3-propanetricaboxylic acid, hexadecane acid, octadecane acid, methanesulfonic acid, ethanesulfonic acid, benzenesulfonic acid, 4-methylbenzenesulfonic acid, cyclohexanesulfamic acid, benzoic acid, 2-hydroxybenzoic acid, 4-aminobenzoic acid, 4-amino-2-hydroxybenzoic acid and the like.

In addition to the active agent, the composition further comprises pharmaceutically acceptable excipients such as fillers, disintegrants, binding agents and lubricants and other additives well known in the art. However, it is an essential feature of the invention that the composition does not contain a wetting agent. By the term "wetting agent" as used herein is meant a substance that reduces the surface tension of a liquid, causing the liquid to spread across or penetrate more easily the surface of a solid.

The term "filler" as used herein refers to agents, which are added to the bulk volume of the powdered drug making up the solid composition. As a result, the size of the solid composition increases, which makes its size suitable for handling. Fillers are only necessary if the dose of drug per solid composition is low and the solid composition would otherwise be too small. Examples of suitable fillers are lactose, sucrose, mannitol, sorbitol, saccharose, starch, pregelatinized starch, microcrystalline cellulose, powdered cellulose, calcium hydrogen phosphate, calcium carbonate and any combinations thereof. In a preferred embodiment the filler is selected from the group consisting of lactose, starch, microcrystalline cellulose and any combinations thereof.

The term "disintegrant" as used herein includes agents, which encourage the break down of the solid composition into small fragments, ideally individual drug particles, when the solid composition is digested. Examples of suitable disintegrants are rice starch, potato starch, maize starch, pregelatinized starch, alginic acid, sodium alginate, sodium starch glycolate, croscarmellose sodium, methylcellulose, powdered cellulose, microcrystalline cellulose, carboxymethylcellulose sodium, crospovidone, colloidal anhydrous silica and any combination thereof. In a preferred embodiment the disintegrant is selected from the group consisting of croscarmellose sodium, microcrystalline cellulose, sodium starch glycolate, crospovidone, maize starch, colloidal anhydrous silica and any combinations thereof.

By the term "binding agent" as used in the present description is meant an agent, which is added to a drug-filler mixture to ensure that granules of the solid composition can be formed with the required mechanical strength. The binding agent holds the solid composition together after it has been compressed, preventing it from breaking down into its component powders during shipping and routine handling. Examples of suitable binding agents are alginic acid, dextrin, pregelatinised maize starch, hydroxypropylmethylcellulose, crosscarmellose sodium, microcrystalline cellulose, methylcellulose, ethylcellulose, hydroxypropyl cellulose, carboxymethylcellulose sodium, Povidone, gelatine and any combinations thereof. In a preferred embodiment the binding agent is selected from the group consisting of Povidone, gelatine, hydroxypropylmethylcellulose, crosscarmellose sodium, microcrystalline cellulose and any combinations thereof.

In the context of the present description, the term "lubricant" refers to an agent that ensures that solid powder does not adhere to the equipment used to press the solid composition during manufacture. Examples of suitable lubricants are stearic acid, polyethylene glycol, calcium stearate, magnesium stearate, glycerol monostearate, glyceryl palmitostearate, hydrogenated vegetable oil, sodium stearyl fumarate, talc and silica (such as colloidal anhydrous silica) and any combinations thereof. In a preferred embodiment the lubricant is selected from the group consisting of magnesium stearate, colloidal anhydrous silica and a combination thereof.

Other additives such as preservatives, flavouring agents and sweeteners may also be present in the composition. However, it is an essential feature of the invention that no wetting agent is present.

It is within the skills of an ordinary practitioner to select a suitable combination comprising a given active compound and a number of additives in order to obtain a suitable solid pharmaceutical composition.

Preferably, the concentration of micronized Nebivolol hydrochloride to be incorporated in the solid composition according to the present invention is in the range of 1-4 %, more preferred in the range of 1.5-3 %, and most preferred in the range of 2.3-2.4 % based on the weight of the composition.

The concentration of the different types of additives may vary depending on the choice of additives, the physical and chemical nature of the specific additive as well as the physical and chemical nature of the composition, in which the additives are combined.

In general, the concentration of the filler is in the range of 70-95 %, more preferred in the range of 80-90 %, and most preferred in the range of 87-89 % based on the weight of the composition. The concentration of the disintegrant generally lies in the range of 3-10 %, more preferred in the range of 5-8 %, and most preferred in the range of 5.9-6.1 % based on the weight of the composition. With regard to the binding agent, the concentration of said agent lies in general in the range of 0.5-6 %, more preferred in the range of 1-5 %, and most preferred in the range of 2-4 % based on the weight of the composition. The concentration of lubricant lies in general in the range of 0.3-1.5 %, more preferred in the range of 0.5-1 %, and most preferred in the range of 0.7-0.9 % based on the weight of the composition.

In the at present most preferred embodiment the content of micronized Nebivolol hydrochloride constitutes 2.3-2.4 %, the content of the filler constitutes 87-89 %, the content of the disintegrant constitutes 5.9-6.1 %, the content of the binding agent constitutes 2-4 % and the content of the lubricant constitutes 0.7-0.9 % by weight of the total weight in the solid pharmaceutical composition.

It is obvious for a person skilled in the art that the composition and concentration of the additives in the composition according to the present invention depends on factors, which may influence the rate of dissolution of the solid composition. It is within the common skills of an ordinary practitioner to design such solid compositions in order to optimize the dissolution characteristics.

The present invention also relates to a method for the manufacture of a tablet comprising the solid pharmaceutical composition according to the present invention, wherein said method comprises the steps of:

(a) preparing a powder by blending micronized Nebivolol or a micronized acid addition salt thereof with part of the filler and part of the disintegrant,
(b) preparing a solution containing the binding agent,
(c) kneading the powder blend from step (a) with the solution containing the binding agent from step (b) and thereby forming a granule,
(d) drying the granule obtained in step (c) until a humidity < 35 % RH is reached,
(e) sieving the dried granules obtained in step (d) in order to obtain granules having a diameter of less than 1.5 mm,
(f) adding the rest of the filler, the rest of the disintegrant and part of the lubricant,
(g) mixing for about 15 minutes,
(h) adding the rest of the lubricant,
(i) mixing for about 3 minutes, and
(j) compressing the granules into tablets.

This method is particularly suitable for the manufacture of a solid composition according to the invention. In step (a) of the method the micronized Nebivolol or the micronized acid addition salt thereof is blended with part of the filler and part of the binding agent. The exact concentration of said three types of agents in the powder blend will depend on the chemical and physical nature of the specific compounds in the blend. A person skilled in the art will understand how to mix the three agents in an appropriate ratio in order to obtain a powder blend for further processing into granules.

The solution obtained in step (b) is usually prepared by dissolving the binding agents in a suitable solvent. The most commonly employed solvent is water, but a person skilled in the art will know in which cases other kinds of solvents advantageously may be employed.

The kneading of the powder blend from step (a) with the solution prepared in step (b) is most often carried out in a high shear mixer or any equivalent type of mixer, and the drying of the granule obtained in step (c) may be performed in a fluid bed. It is within the skills of an ordinary practitioner to obtain a granule having a humidity < 35 % RH.

The sieving of the dried granules obtained in step (d) may be performed by using any suitable sieves known within the art. The mixing in step (g) and step (i) is performed in a cone mixer or any other equivalent type of mixer. A person skilled in the art will know how to compress the granules into tablets in step (j).

The present invention also relates to the use of the solid pharmaceutical composition according to the invention in the manufacture of a tablet for use as a β-adrenergic receptor-blocking drug or for use in the treatment of essential hypertension or in the treatment of chronic heart failure.

It is within the knowledge of a skilled person to design a tablet having a suitable concentration of Nebivolol depending on whether the tablet is designed for administration one, two or three times a day.

The present invention is more specifically explained by the following examples. However, it should be understood that the scope of the present invention is not limited by the examples in any manner. It will be appreciated by any person skilled in the art that the present invention includes any variation within the technical concept of the present invention.

Examples

1. Determination of the particle size of Nebivolol or of the micronized acid addition salt thereof

The particles are measured at a Malvern Master sizer having the following specifications:

Range lens 300 RF mm, with backscatter detector
Analysis model: Polydisperse
Presentation: Fraunhofer

Method: Approximately 80 ml of Migliol/Tegosoft is added to the stirring chamber. 50 mg API in 10 ml of Migliol or in 10 ml of Tegosoft CT is then dispersed therein. The dispersion is treated in an ultra sonic bath for 3 minutes, and then added to the stirring chamber drop by drop until the obscuration is 10-20. Then the measuring is started.

For the sample of Nebivolol particles or particles of a micronized acid addition salt thereof to be acceptable for use in the solid pharmaceutical composition according to the invention, the results of the determination of the particle size should fulfil the following specifications:

$$D(v,0.5) < 10 \ \mu m$$

$$D(v,0.9) < 40 \ \mu m$$

2. Dissolution test

For the dissolution test a paddle apparatus as described in the European Pharmacopoeia with a rotation speed set at 50 ± 2 revolution per minute was used. 800 ml simulated gastric fluid (SGF) was used as medium at a fixed temperature of 37°C ± 0.5°C.

The simulated gastric fluid (SGF) was prepared by dissolving 4.0 g sodium chloride and 6.4 g pepsin in 1000 ml of water in a 2000 ml measuring flask. Then 160 ml 1 M hydrochloric acid was added and the measuring flask was filled up with water. The final pH of the solution should be about 1.2.

Method: One tablet comprising Nebivolol hydrochloride was placed in each of the 6 vessels. 10 ml from each vessel was sampled after: 5, 10, 15, 20, 30, 45 min. The withdrawn sample is centrifuged and then analysed according to the HPLC method having the following characteristics:

| | | |
|---|---|---|
| Column: | Synergi polar RP, 75 x 3 mm, 4 μm | |
| Mobile phase: | 0.025 M $KH_2PO_4$ pH 3* | 65 |
| | Acetonitrile | 35 |

(continued)

| | |
|---|---|
| Flow : | 1 ml/min. |
| Injection volume: | 20 $\mu$l |
| Detection: | UV-220 nm |
| Temperature: | 40°C |
| Runtime: | approx. 5 min. |

Preparation of buffer: 3.4 g $KH_2PO_4$ was dissolved in 1000 ml of water and the pH value of the solution was adjusted to 3. The mobile phase was buffer and acetonitrile. Then the solution was tested.

Preparation of standard solution: 33 mg Nebivolol hydrochloride standard was dissolved in 10 ml methanol in a 100 ml volumetric flask, which then was filled up with mobile phase. This solution was diluted 50 times using mobile phase.

Calculation of Nebivolol hydrochloride dissolved is expressed as Nebivolol, and the correction factor is incorporated in the % standard:

Withdrawn sample (5 min.):

$$\frac{\text{Area test x weighed st. x \% standard x 800}}{\text{Area st. x 5(decl. cont.) x 50 x 100}}$$

The next calculations are corrected for withdrawn ml medium and amount of API.

2. withdrawn sample (10 min.):

$$\frac{\text{Area test x weighed st. x \% standard x (800-10)}}{\text{Area st. x 5(decl. cont.) x 50 x 100}} + \frac{10 \text{ x \% dissolve. 5 min.}}{800}$$

3. withdrawn sample (15 min.):

$$\frac{\text{Area test x weighed st. x \% standard x (800-20)}}{\text{Area st. x 5(decl. cont.) x 50 x 100}} + \frac{10 \text{ x \% dissolv. 5 min.}}{800} +$$

$$\frac{10 \text{ x \% dissolv. 10 min.}}{(800 - 10)}$$

4. withdrawn sample (20 min.):

$$\frac{\text{Area test x weighed st. x \% standard x (800-30)}}{\text{Area st. x 5(decl. cont.) x 50 x 100}} + \frac{10 \text{ x \% dissolv. 5 min.}}{800} +$$

$$\frac{10 \text{ x \% dissolv.10 min.}}{(800-10)} + \frac{10 \text{ x \% dissolv. 15 min}}{(800-20)}$$

5. withdrawn sample (30 min.):

$$\frac{\text{Area test x weighed st. x \% standard x (800-40)}}{\text{Area st. x 5(decl. cont.) x 50 x 100}} + \frac{10 \text{ x \% dissolv. 5 min.}}{800} +$$

$$\frac{10 \text{ x \% dissolv.10 min.}}{(800-10)} + \frac{10 \text{ x \% dissolv. 15 min}}{(800-20)} + \frac{10 \text{ x \% dissolv. 20 min.}}{(800-30)}$$

6. withdrawn sample (45 min.):

$$\frac{\text{Area test x weighed st. x \% standard x (800-50)}}{\text{Area st. x 5(decl. cont.) x 50 x 100}} + \frac{10 \text{ x \% dissolv. 5 min.}}{800} +$$

$$\frac{10 \text{ x \% dissolv.10 min.}}{(800-10)} + \frac{10 \text{ x \% dissolv. 15 min}}{(800-20)} + \frac{10 \text{ x \% dissolv. 20 min.}}{(800-30)} +$$

$$\frac{10 \text{ x \% dissolv. 30 min.}}{(800-40)}$$

3. Compositions according to the present invention

Preferred tablets according to the present invention was prepared using the following ingredients:

|  | Tablet A | Tablet B | Tablet C | Tablet D |
|---|---|---|---|---|
| I: |  |  |  |  |
| Micronized Nebivolol hydrochloride | 5.45 mg | 5.45 mg | 5.45 mg | 5.45 mg |
| Croscarmellose Sodium | 6.90 mg | 6.90 mg |  |  |
| Sodium Starch Glycolate |  |  | 6.90 mg |  |
| Crospovidone |  |  |  | 6.90 mg |
| Lactose | 142.00 mg | 142.00 mg | 142.00 mg | 142.00 mg |
| Maize Starch | 46.00 mg | 46.00 mg | 46.00 mg | 46.00 mg |
| II: |  |  |  |  |
| Povidone K 30 | 9.40 mg |  |  |  |
| Gelatine |  | 4.60 mg |  |  |
| Hydroxypropylmethylcellulose 5 cps |  |  | 4.60 mg | 4.60 mg |
| III: |  |  |  |  |

(continued)

|  | Tablet A | Tablet B | Tablet C | Tablet D |
|---|---|---|---|---|
| Microcrystalline Cellulose | 16.31 mg | 16.31 mg | 16.31 mg | 16.31 mg |
| Croscarmellose Sodium | 6.90 mg | 6.90 mg | | |
| Sodium Starch Glycolate | | | 6.90 mg | |
| Crospovidone | | | | 6.90 mg |
| Colloidal anhydrous silica | 0.65 mg | 0.65 mg | 0.65 mg | 0.65 mg |
| IV: | | | | |
| Magnesium stearate | 1.15 mg | 1.15 mg | 1.15 mg | 1.15 mg |
| Tablet weight | 235 mg | 230 mg | 230 mg | 230 mg |

The tablets were prepared by a manufacturing procedure as described below:

A powder blend was prepared by blending the group I agents in a high shear mixer. A solution containing the group II agents was prepared and then the powder blend of group I agents was kneaded with the solution of group II agents in a high shear mixer, and thereby forming a granule. The granule was dried in a fluid bed until a relative humidity < 35 % RH was reached, and afterwards the dried granule was sieved in order to obtain granules having a diameter of less than 1.5 mm. The group III agents were then added, and mixed in a cone mixer for 15 minutes. The group IV agent was then added to the granule and mixed in a cone mixer for 3 minutes.

Finally, the granule was transferred to a tablet press and the granules were compressed into tablets using a punch, which was 9 mm round and was flat faced with bevelled edges. The hardness of the resulting tablets were measured to > 45 N. And the disintegrations experiments showed a disintegration in the range of 2 - 5 minutes.

4. Results of dissolution test

The results with respect to the dissolution test performed on the tablets A, B, C and D as described above under item 2 is given in the table below.

| Time (minutes) | % dissolved | | | |
|---|---|---|---|---|
| | Tablet A | Tablet B | Tablet C | Tablet D |
| 5 | 62 | 67 | 62 | 72 |
| 10 | 82 | 83 | 82 | 89 |
| 15 | 88 | 88 | 88 | 93 |
| 20 | 91 | 91 | 91 | 94 |
| 30 | 92 | 92 | 95 | 95 |
| 45 | 94 | 92 | 96 | 96 |

As can be seen from the results in the table, the tablets A, B, C and D showed a dissolution of 82 %, 83 %, 82 % and 89 %, respectively, within 10 minutes. Hence, all of the tablets clearly satisfy the requirements as claimed stating that the composition according to the invention must show a dissolution of at least 75 % within 45 minutes.

**Claims**

1. A solid pharmaceutical composition comprising micronized Nebivolol or a pharmaceutically acceptable micronized acid addition salt thereof as the active agent, a filler, a disintegrant, a binding agent and a lubricant, wherein the dissolution of said solid composition is at least 75 % after 45 minutes.

2. A solid composition according to claim 1, wherein the volume median diameter of the particles of the micronized Nebivolol or the pharmaceutically acceptable micronized acid addition salt thereof is < 10 $\mu$m.

3. A solid pharmaceutical composition according to any of the claims 1 or 2, wherein the active agent is micronized Nebivolol hydrochloride.

4. A solid pharmaceutical composition according to any of the claims 1 to 3, wherein the filler is selected from the group consisting of lactose, starch, microcrystalline cellulose and any combinations thereof.

5. A solid pharmaceutical composition according to any of the claims 1 to 4, wherein the disintegrant is selected from the group consisting of croscarmellose sodium, microcrystalline cellulose, sodium starch glycolate, crospovidone, maize starch, colloidal anhydrous silica and any combinations thereof.

6. A solid pharmaceutical composition according to any of the claims 1 to 5, wherein the binding agent is selected from the group consisting of Povidone, gelatine, hydroxypropylmethylcellulose, crosscarmellose sodium, microcrystalline cellulose and any combinations thereof.

7. A solid pharmaceutical composition according to any of the claims 1 to 6, wherein the lubricant is selected from the group consisting of magnesium stearate, colloidal anhydrous silica and any combinations thereof.

8. A solid pharmaceutical composition according to any of the claims 1 to 7, wherein the content of micronized Nebivolol hydrochloride constitutes 2.3-2.4 %, the content of the filler constitutes 87-89 %, the content of the disintegrant constitutes 5.9-6.1 %, the content of the binding agent constitutes 2-4 % and the content of the lubricant constitutes 0.7-0.9 % based on the weight of the composition.

9. A method for the manufacture of a tablet comprising the solid pharmaceutical composition according to any of the claims 1 to 8, wherein said method comprises the steps of:

(a) preparing a powder by blending micronized Nebivolol or a micronized acid addition salt thereof with part of the filler and part of the disintegrant,
(b) preparing a solution containing the binding agent,
(c) kneading the powder blend from step (a) with the solution containing the binding agent from step (b) and thereby forming a granule,
(d) drying the granule obtained in step (c) until a humidity < 35 % RH is reached,
(e) sieving the dried granules obtained in step (d) in order to obtain granules having a diameter of less than 1.5 mm,
(f) adding the rest of the filler, the rest of the disintegrant and part of the lubricant,
(g) mixing for about 15 minutes,
(h) adding the rest of the lubricant,
(i) mixing for about 3 minutes, and
(j) compressing the granules into tablets.

10. Use of the solid pharmaceutical composition according to any of the claims 1 to 8 in the manufacture of a tablet for use as a β-adrenergic receptor-blocking drug or for use in the treatment of essential hypertension or in the treatment of chronic heart failure.

**European Patent Office**

**EUROPEAN SEARCH REPORT**

Application Number

EP 06 38 8023

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/025070 A (TORRENT PHARMACEUTICALS LIMITED; SHETH, RAKESH; ATTANTI, SRINIVASARAO,) 9 March 2006 (2006-03-09)<br>* claims 1-22 *<br>* page 28, line 8 - page 32, line 3 *<br>* tables 1-19 *<br>* examples 1-21 *<br>* claims 20-23 *<br>----- | 1-10 | INV.<br>A61K31/353<br>A61K9/16 |
| X | WO 95/22325 A (JANSSEN PHARMACEUTICA N.V; JANS, EUGEEN; SMANS, GUIDO, FRANCISCUS; GIL) 24 August 1995 (1995-08-24)<br>* claims 1-10 *<br>* examples 1-4 *<br>* page 1, line 10 - page 6, line 4 *<br>----- | 1-10 | |
| X | US 2005/272810 A1 (DAVIS ERIC ET AL) 8 December 2005 (2005-12-08)<br>* paragraph [0147] - paragraph [0182] *<br>----- | 1-10 | |
| | | | **TECHNICAL FIELDS SEARCHED (IPC)**<br><br>A61K |

The present search report has been drawn up for all claims

| Place of search | Date of completion of the search | Examiner |
|---|---|---|
| Munich | 8 September 2006 | Schifferer, Hermann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 06 38 8023

This annex lists the patent family members relating to the patent documents cited in the above-mentioned European search report.
The members are as contained in the European Patent Office EDP file on
The European Patent Office is in no way liable for these particulars which are merely given for the purpose of information.

08-09-2006

| Patent document cited in search report | | Publication date | Patent family member(s) | | Publication date |
|---|---|---|---|---|---|
| WO 2006025070 | A | 09-03-2006 | NONE | | |
| WO 9522325 | A | 24-08-1995 | AT | 212547 T | 15-02-2002 |
| | | | AU | 688860 B2 | 19-03-1998 |
| | | | AU | 1706895 A | 04-09-1995 |
| | | | BR | 9506828 A | 30-09-1997 |
| | | | CA | 2182582 A1 | 24-08-1995 |
| | | | CN | 1140991 A | 22-01-1997 |
| | | | CY | 2329 A | 06-02-2004 |
| | | | CZ | 9602346 A3 | 13-11-1996 |
| | | | DE | 69525241 D1 | 14-03-2002 |
| | | | DE | 69525241 T2 | 29-08-2002 |
| | | | DK | 744946 T3 | 06-05-2002 |
| | | | EP | 0744946 A1 | 04-12-1996 |
| | | | ES | 2171533 T3 | 16-09-2002 |
| | | | FI | 963227 A | 16-08-1996 |
| | | | HU | 75332 A2 | 28-05-1997 |
| | | | IL | 112659 A | 20-06-1999 |
| | | | JP | 9508913 T | 09-09-1997 |
| | | | NO | 963439 A | 16-08-1996 |
| | | | NZ | 279822 A | 24-04-1997 |
| | | | PL | 315973 A1 | 23-12-1996 |
| | | | PT | 744946 T | 31-07-2002 |
| | | | RU | 2137473 C1 | 20-09-1999 |
| | | | SG | 47487 A1 | 17-04-1998 |
| | | | SK | 105896 A3 | 07-05-1997 |
| | | | ZA | 9501294 A | 16-08-1996 |
| US 2005272810 | A1 | 08-12-2005 | WO | 2005117858 A2 | 15-12-2005 |

**EP 1 839 658 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0145067 B1
- EP 0334429 B1
- EP 0744946 B1